(19) Europäisches Patentamt
European Patent Office
Office européen des brevets

(11) Veröffentlichungsnummer: **0 091 596 B1**

# (12) EUROPÄISCHE PATENTSCHRIFT

(45) Veröffentlichungstag der Patentschrift: **11.09.91**

(21) Anmeldenummer: **83103022.6**

(22) Anmeldetag: **26.03.83**

(51) Int. Cl.[5]: **C07D 235/02**, C07D 401/04, C07D 233/74, C07D 233/86, C07D 233/76, C07D 233/78, A01N 43/50, A01N 43/52, A01N 43/40

## (54) Neue Hydantoine, ihre Herstellung und Verwendung.

(30) Priorität: **08.04.82 DE 3213140**
**16.10.82 DE 3238447**
**14.01.83 DE 3301008**

(43) Veröffentlichungstag der Anmeldung:
**19.10.83 Patentblatt 83/42**

(45) Bekanntmachung des Hinweises auf die Patenterteilung:
**11.09.91 Patentblatt 91/37**

(84) Benannte Vertragsstaaten:
**AT BE CH DE FR GB IT LI NL SE**

(56) Entgegenhaltungen:
**EP-A- 0 001 813**
**DE-A- 2 102 605**
**DE-A- 2 126 187**
**GB-A- 997 037**

(73) Patentinhaber: **SHELL INTERNATIONALE RESEARCH MAATSCHAPPIJ B.V.**
**Carel van Bylandtlaan 30**
**NL-2596 HR Den Haag(NL)**

(72) Erfinder: **Schröder, Ludwig, Dr. Dipl.-Chem.**
**Frankenstrasse 7**
**W-6507 Ingelheim(DE)**
Erfinder: **Stransky, Werner, Dr. Dipl.-Chem.**
**Im Hippel 24**
**W-6535 Gau-Algesheim(DE)**
Erfinder: **Mengel, Rudolf, Dr. Dipl.-Chem.**
**Schützenpfad 22**
**W-6507 Ingelheim(DE)**
Erfinder: **Lust, Sigmund, Dr.**
**Klappacher Strasse 2f**
**W-6100 Darmstadt(DE)**
Erfinder: **Linden, Gerbert, Dr. Dipl.-Landwirt**
**Turnierstrasse 44**
**W-6507 Ingelheim(DE)**
Erfinder: **Raddatz, Erich, Dr.**
**Carrera 1-Oe No. 5-265**
**Cali(CO)**

**EP 0 091 596 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Schneider, Gerhard, Dipl.-Bio.**
**Schleifmühlenweg 7a**
**W-6109 Mühltal 1(DE)**

(74) Vertreter: **Bennett, David Arthur Horder et al**
**4, York Road**
**London SE1 7NA(GB)**

## Beschreibung

Die Erfindung betrifft neue Hydantoine, ihre Herstellung nach an sich bekannten Verfahren und ihre Verwendung bei der Bekämpfung unerwünschten Pflanzenwachstums.

Die Europäische Patentanmeldung mit der Veröffentlichungsnummer 0 001 813 Al offenbart gewisse Imidazolidinderivate, die nützliche therapeutische Eigenschaften aufweisen. Die deutsche Offenlegungsschrift Nr 2102605 offenbart gewisse Thiohydantoinderivate als herbizide Mittel. Die deutsche Offenlegungsschrift Nr 2 126 187 offenbart gewisse Hydantoinderivate und Imidazolidinonderivate als Mittel gegen das Ausbleichen von Farbfotografien.

Die neuen Verbindungen der vorliegenden Erfindung haben die Formel

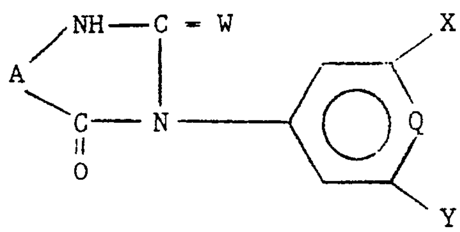

(I),

in der

A für einen gegebenenfalls ein-oder mehrfach verbrückten Cycloalkanrest der Formel

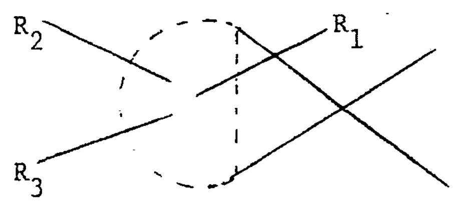

(II)

mit 5 bis 10 C-Atomen oder für den Rest

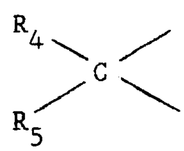

,

Q für CH oder N,

$R_1$, $R_2$ und $R_3$, die gleich oder verschieden sein können, für Wasserstoff, für geradkettiges oder verzweigtes $C_1$-$C_4$-Alkyl oder für garadkettiges oder verzweigtes $C_3$-$C_4$-Alkenyl,

$R_4$ und $R_5$, die gleich oder verschieden sein können, für $C_1$-$C_4$-Alkyl, das auch durch $C_1$-$C_4$-Alkyl-O- oder $C_1$-$C_4$-Alkyl-S-oder eine gegebenenfalls ein- oder mehrfach durch $C_1$-$C_4$-Alkyl, $C_1$-$C_4$-Alkoxy, Trifluoromethyl und Halogen substituierte Phenyl-O-oder Phenyl-S-Gruppe substituiert sein kann, für $C_2$-$C_4$-Alkenyl, für $C_3$-$C_6$-Cycloalkyl, das auch niederalkylsubsituiert sein kann, für gegebenenfalls ein- oder mehrfach durch $C_1$-$C_4$-Alkyl, $C_1$-$C_4$-Alkoxy, Trifluoromethyl und Halogen substituierte Phenyl oder Benzyl, mit der Massgabe, dass wenn $R_4$ nichtsubstituiertes Methyl ist, $R_5$ nicht für Methyl oder Ethyl steht,

W für Sauerstoff, und

X und Y, die gleich oder verschieden sein können, für Halogen, $C_1$-$C_4$-Alkyl, $C_1$-$C_4$-Alkoxy oder Trifluormethyl, X ausserdem auch für Wasserstoff

steht, mit der Massgabe, dass wenn A eine nichtsubstituierte Cycloalkylgruppe und Q CH ist, X nicht für Wasserstoff stehen kann.

Diese Verbindungen zeichnen sich durch eine starke Wirkung gegen Unkräuter and Ungräser aus und

können in zahlreichen Kulturen als selektive Herbizide eingesetzt werden.

In den obigen Definitionen ist unter Halogen Fluor, Chlor, Brom oder Jod (bevorzugt Chlor) zu verstehen. X steht vor allem für Wasserstoff, Chlor, Brom, Methyl, Methoxy und Trifluormethyl, Y vor allem für Chlor, Brom, Methyl, Methoxy und Trifluormethyl. Die Niederalkyl- und Niederalkoxyreste sowie die $C_1$-$C_4$-Alkyl- und -Alkoxyreste sind bzw. umfassen Methyl, Ethyl, n-Propyl, i-Propyl, n-Butyl, i-Butyl, s-Butyl und t-Butyl. $R_4$ und $R_5$ sind vor allem n-Propyl und i-Propyl. Als Alkenyl ist besonders Allyl zu nennen.

Soweit $R_4$ und/oder $R_5$ substituierte Phenyl- oder Benzylreste bedeuten oder enthalten, sind die Substituenten ein oder mehrere $C_1$-$C_4$-Alkyl- oder $C_1$-$C_4$-Alkoxyreste, Trifluormethyl oder Halogen, gegebenenfalls in Mischung. Dabei sind Methyl, Methoxy, Chlor und Brom hervorzuheben. Die Gruppe A leitet sich vor allem von Cyclohexan, Cyclopentan oder ihren ein- bis dreifach niederalkyl-, insbesondere methylsubstituierten Homologen ab, etwa Menthan, oder von bi- oder tricyclischen, gegebenenfalls ein- bis dreifach niederalkyl-, insbesondere methyl-substituierten Cycloalkanen, etwa Caran, Pinan.

Die Verbindungen der Formel I können nach an sich bekannten Methoden hergestellt werden, indem man

a) zur Herstellung solcher Verbindungen, in denen Q CH ist, eine Carbonsäure der Formel

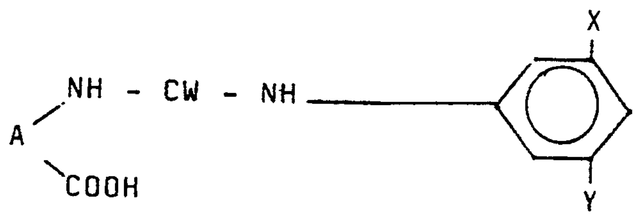

(III),

worin A, W, X und Y die obige Bedeutung haben, in Gegenwart einer starken Mineralsäure cyclisiert oder daß man

b) zur Herstellung solcher Verbindungen, in denen Q CH ist, einen Ester der Formel

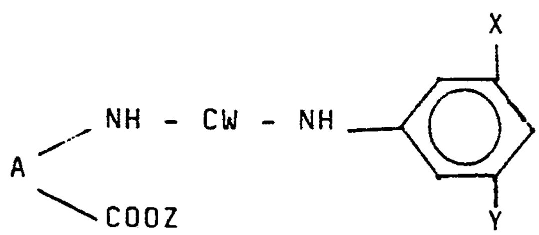

(IV),

worin A, W, X und Y die obige Bedeutung haben und Z für einen geradkettigen oder verzweigten, gegebenenfalls substituierten aliphatischen $C_1$-$C_{20}$- Rest oder einen gegebenenfalls substituierten araliphatischen Rest steht, in Gegenwart einer Base cyclisiert oder daß man

c) zur Herstellung solcher Verbindungen, in denen Q CH ist, eine Aminoverbindung der Formel

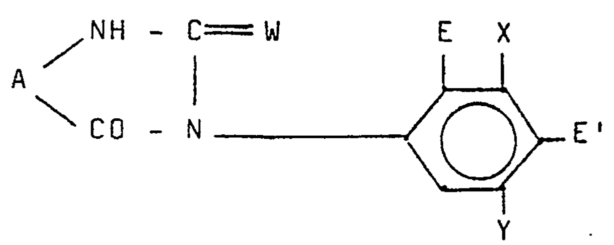

(V),

worin A, W, X und Y die obige Bedeutung haben und E und E' für Wasserstoff oder $NH_2$ stehen, wobei mindestens einer dieser beiden Reste $NH_2$ bedeutet, zur Entfernung von E und/oder E' entaminiert oder daß man

d) zur Herstellung solcher Verbindungen der Formel I, in denen Q für N steht, ein Hydantoin der Formel

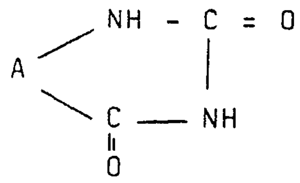

(VI)

worin A die obige Bedeutung hat, mit einem Pyridin der Formel

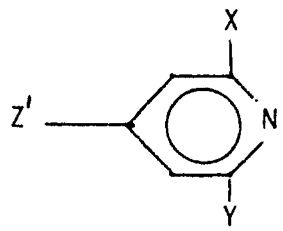

(VII)

worin X und Y die obige Bedeutung haben und Z' für $NO_2$ oder Halogen steht, unter Zugabe einer basischen Verbindung (z.B. Kalium- oder Natriumcarbonat, Kalium-Natrium- oder Calciumhydroxid) bei Temperaturen zwischen etwa 0 °C und etwa 80 °C umsetzt.

Die Cyclisierung der Säure gemäß Verfahren a) erfolgt zweckmäßig in wäßriger oder alkoholischer Lösung in Gegenwart einer Säure wie Salzsäure, Schwefelsäure bei erhöhter Temperatur. Am einfachsten ist es, die Mischung einige Zeit auf Siedetemperatur zu erhitzen. Die Cyclisierung der Ester gemäß Verfahren b) erfolgt bevorzugt in einem Alkohol oder einem anderen organischen Lösungsmittel, beispielsweise Dioxan. Als Base wird bevorzugt eine tertiäre organische Base verwendet, z.B. Triethylamin, Tripropylamin. Das Reaktionsgemisch wird erwärmt, im allgemeinne einige Stunden auf Siedetemperatur.
Im Verfahren c)erfolgt die Entfernung der Aminogruppe(n) in an sich bekannter Weise über die Diazonium-Verbindungen, indem man z.B. die Diazotierung in Gegenwart von siedendem Ethanol vornimmt (Houben-Weyl, Bd. 10/3 (1965), Seite 116 ff) oder die Suspension bzw. Lösung des Diazoniumsalzes in eine wäßrige Lösung von unterphosphoriger Säure einträgt (a.a.O, S. 131 ff.) oder die Diazotierung mit Alkylnitriten in Gegenwart von Derivaten der Ameisensäure, etwa Dimethylformamid, durchführt (a.a.O, S. 137 ff.).

Verfahren d) kann durch Zugabe eines Phasentransferkatalysators (z.B. Kronenether, Tetraalkylammoniumsalze, Tetraalkylphosphoniumsalze) günstig beeinflußt werden.

Die erfindungsgemäßen Verbindungen können z.T. in Form von cis-/trans-Isomeren und/oder in enantiomeren Formen vorliegen. Die Zuordnung zur cis- bzw. trans-Reihe ist nach Cahn-Ingold-Prelog vorgenommen worden. Erfindungsgemäß erhaltene Gemische geometrischer Isomerer können gewünschtenfalls nachträglich aufgetrennt werden, z.B. durch fraktionierte Kristallisation, ebenso Racemate.

Verwendet man zur Herstellung der Ausgangsstoffe (VIII, X) unsymmetrisch substituierte Ketone, können je nach den Synthesebedingungen unterschiedliche geometrische Isomere gebildet werden. Entsprechend werden daraus schließlich die isomeren Cycloalkan-spirohydantoine I gebildet (vgl. z.B. L. Hoyer, Chem.Ber. 83, S. 491 (1950)).

Die Ausgangsstoffe III bis VII sind bekannt oder können nach üblichen Verfahren analog bekannten Verbindungen hergestellt werden.

Zur Herstellung der Verbindungen der Formel III setzt man beispielsweise eine 1-Aminocycloalkancarbonsäure der Formel

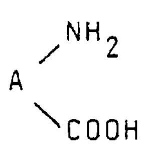

(VIII),

worin A die obige Bedeutung hat, mit einem Isocyanat der Formel

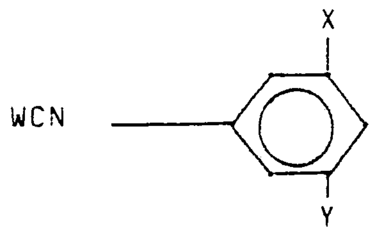

(IX),

worin W, X und Y die obige Bedeutung haben, in wäßriger oder alkoholischer Lösung bei niedrigen Temperaturen, vorzugsweise 0 - 10° C, in Gegenwart einer basischen Substanz (z.B. Natronlauge, Kalilauge, Kaliumcarbonat, Natriummethylat) um und fällt mit einer geeigneten Säure (z.B. Salzsäure, Schefelsäure, Essigsäure) die Verbindung der Formel III aus.

Die Ausgangsstoffe der Formel IV können durch Umsetzung von Estern der Formel

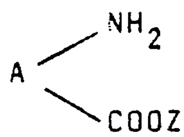

(X),

worin A und Z die obige Bedeutung haben, mit einem Isocyanat der Formel IX erhalten werden. Man bringt die Reaktionspartner IX und X in einem inerten Lösungsmittel /z.B. Ether, Methylenchlorid, Toluol, Essigester) bei niedrigen Temperaturen, vorzugsweise 10 - 20° C, zur Reaktion.

Der Rest Z bedeutet im allgemeinen einen niederen bis mittleren Alkylrest ($C_1$-$C_{12}$) oder einen gegebenenfalls substituierten Benzylrest. Seine Art ist im allgemeinen unkritisch, d.h. sofern er nicht wegen ungünstiger Struktur oder wegen reaktionsfähiger Substituenten zu Nebenreaktionen oder einer Hemmung der Reaktion führt.

Die Ausgangsstoffe für das Verfahren c) können z.B. wie folgt erhalten werden:
Man setzt ein Spirohydantoin der Formel

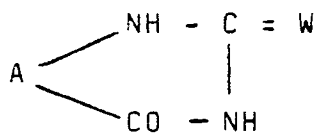

(XI),

worin A und W die obige Bedeutung haben, mit einem Halogennitrobenzol der Formel

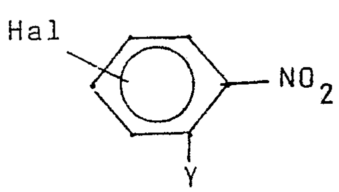

oder

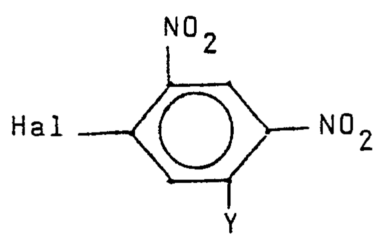

(XIIa) (XIIb)

(Hal: Fluor oder Chlor in 2- oder 4-Stellung, Y in der obigen Bedeutung) in Gegenwart einer Base (z.B. $K_2CO_3$, $Na_2CO_3$, KOH, NaOH, etc.)
in einem geeigneten Lösungsmittel (z.B. Dimethylsulfoxid, Acetonitril, Aceton), gegebenenfalls unter Zusatz eines Phasentransferkatalysators (Kronenether, Tetraalkylammoniumsalze, Tetraalkylphosphoniumsalze) bei Temperaturen zwischen 0° C und 150° C zu Verbindungen der Formel

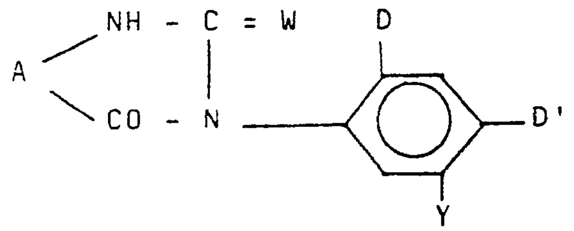

(XIII)

(A, W, Y in der obigen Bedeutung; D, D' H oder $NO_2$, jedoch mindestens eins von beiden $NO_2$) um.

Die Verbindungen XIII werden zu den entsprechenden Aminoverbindungen der Formel

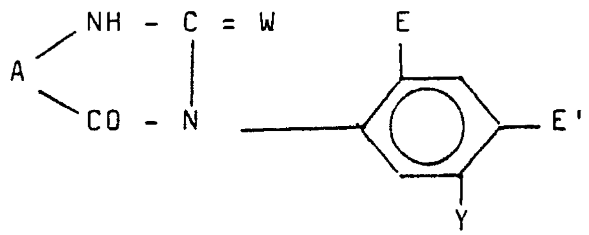

(Va)

(A, W, Y in der obigen Bedeutung; E, E' H oder $NH_2$, jedoch mindestens eines von beiden $NH_2$) reduziert. Diese Aminoverbindungen selbst oder ihr Halogenierungsprodukt (nach Halogenierung mit z.B. $Cl_2$, $SO_2Cl_2$, $Br_2$), Verbindungen der Formel V mit X gleich Chlor oder Brom werden dann gemäß Verfahren c) entaminiert.

Die erfindungsgemäßen Verbindungen zeichnen sich durch eine starke Wirkung gegen zahlreiche, insbesondere monokotyle Unkräuter aus. Die Anwendung erfolgt bevorzugt vor dem Auflaufen. Die gute Verträglichkeit der erfindungsgemäßen Wirkstoffe ermöglicht den Einsatz in zahlreichen Kulturen, z.B. in Soja, Mais, Reis, Baumwolle, Gerste, Rüben, Kartoffeln, Tomaten, Zwiebeln.

Die Aufwandmengen können je nach Substanz, Unkraut und äußeren Bedingungen zwischen 0,1 und 10 kg/ha, insbesondere zwischen 0,3 und 3kg/ha schwanken.

Häufig erweist es sich als vorteilhaft, Kombinationen von Verbindungen der Formel 1 und bekannten Herbiziden anzuwenden. Zu nennen sind hier Kombinationen mit Harnstoff-Derivaten (z.B. Chlortoluron), Triazin-Derivaten (z.B. Atrazin, Simazin), Dinitroanilin-Derivaten (z.B. Trifluralin), Chloracetanilid-Derivaten (z.B. Alachlor), Thiocarbamaten (z.B. Benthiocarb), Diphenylether (z.B. Acifluorfen).

Die Wirkstoffe der Formel I können für die Anwendung in gebräuchlicher Weise zu üblichen Formulierungen verarbeitet werden, z.B. zu Granulaten, Stäubemitteln, Suspensionspulvern bzw. -konzentraten, wasserdispergierbaren Granulaten.

Diese Formulierungen werden hergestellt durch Vermischen bzw. Vermahlen der Wirkstoffe mit Streckmitteln, z.B. Lösungsmitteln und/oder festen Trägerstoffen, gegebenenfalls unter Zusatz oberflächenaktiver Mittel (Emulgatoren, Dispergiermitteln) und/oder stabilisierender und/oder schaumverhindernder Mittel sowie gegebenenfalls weiterer Zusätze.

Als Lösungsmittel wird Wasser bevorzugt; als feste Trägerstoffe eignen sich beispielsweise Gesteinsmehle (z.B. Kaoline, Tonerden, Talkum, Kreide, Quarz, hochdisperse Kieselsäure, Aluminiumoxid, Silikate). Für Granulate geeignete Träger sind einerseits gebrochene und fraktionierte Gesteine (z.B. Calcit, Marmor, Bims), andererseits Granulate aus organischem Material (z.B. aus Sägemehl, Kokosnußschalen, Maiskolben).

Als Emulgatoren eignen sich nichtionogene und anionische Verbindungen, etwa Polyoxyethylen-Fettsäure-Ester, Polyoxyethylen-Fettalkohol-Ether, z.B. Alkylarylpolyglykolether, Alkylsulfonate, Alkylsulfate, Arylsulfonate und Eiweißhydrolysate.

Als Dispergiermittel kommen z.B. Sulfitablaugen aus der Holzverarbeitung oder Methylzellulose in Betracht, als schaumverhindernde Mittel verzweigte höhere Alkohole.

Aus den konzentrierten Zubereitungen, die im allgemeinen zwischen 0,1 und 95 Gewichtsprozent, vorzugsweise zwischen 0,5 und 90 Gewichtsprozent Wirkstoff enthalten, werden gegebenenfalls durch Verdünnen mit Wasser Spritz- oder Gießbrühen gewünschter Konzentration hergestellt.

Die Anwendung erfolgt je nach der Zubereitung durch Gießen, Spritzen, Streuen oder Stäuben.

Formulierungsbeispiele

a) Suspensionspulver

25 Gew.-% einer Verbindung der Formel I
55 Gew.-% Kaolin
10 Gew.-% kolloidale Kieselsäure
9 Gew.-% Ligninsulfonat
1 Gew.-% Natriumtetrapropylenbenzolsulfonat

b) Suspensionspulver

80 Gew.-% einer Verbindung der Formel I
8 Gew.-% Calciumligninsulfonat
5 Gew.-% kolloidale Kieselsäure
5 Gew.-% Natriumsulfat
2 Gew.-% Natriumdiisobutylnaphthalinsulfonat

Beispiel 1

Cyclohexan-5'-spiro-3'-(3,5-dichlorphenyl)-hydantoin

171 g (1 mol) 1-Amino-cyclohexan-carbonsäure-ethyl-ester, hergestellt durch Veresterung (mit HCl/EtOH) von 1-Amino-cyclohexan-carbonsäure, werden in 500 ml Ether gelöst. Man läßt unter Eiskühlung eine Lösung von 188,5 g (1 mol) 3,5-Dichlorphenylisocyanat in 500 ml Ether innerhalb von 15 Minuten einfließen. Nach beendeter Zugabe wird noch 30 Minuten bei Raumtemperatur gerührt und danach abgesaugt.

Der Nutschenrückstand wird nun in 500 ml Ethanol suspendiert und nach Zugabe von 10,1 g (0,1 mol) Triethylamin 3 Stunden unter Rückfluß gekocht. Danach gießt man auf 2 Ltr. Eiswasser und saugt den entstandenen Niederschlag ab. Man wäscht gründlich mit Wasser und trocknet im Umluftschrank.

Man erhält ca. 280 g (89 % d.Th.) der Titelverbindung. Fp. 210° - 211° C (aus Ethanol).

Die stereochemische Zuordnung (cis bzw. trans) erfolgte entsprechend den Regeln nach Cahn-Ingold-Prelog).

Ausgangsstoffe der Formel III und IV:

| Nr. | A | W | X | Y | Z | Fp. [°C] |
|---|---|---|---|---|---|---|
| 1 | 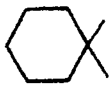 | O | Cl | Cl | $C_2H_5$ | 178-179 |
| 2 | 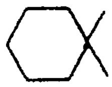 | O | Cl | Cl | H | 195-197 |
| 3 | 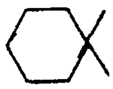 | O | $CH_3$ | $CH_3$ | H | 186-188 |
| 4 | 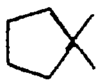 | O | Cl | Cl | H | 163-164 |
| 5 | 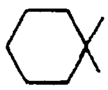 | O | H | $CF_3$ | $C_2H_5$ | 131-132 |
| 6 | 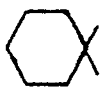 | O | H | $CH_3$ | $C_2H_5$ | 135-136 |
| 7 | 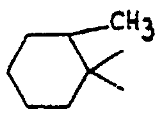 (2-cis) | O | Cl | Cl | H | 175-177 |
| 8 | 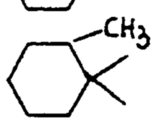 (2-cis) | O | Cl | Cl | $C_2H_5$ | 188-189 |
| 9 | 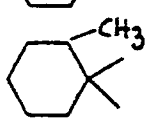 (2-cis) | O | $CH_3$ | $CH_3$ | H | 188-190 |
| 10 | 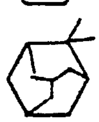 | O | Cl | Cl | H | 238-240 |
| 11 | 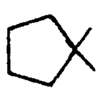 | O | Cl | Cl | $C_2H_5$ | 186-187 |
| 12 | 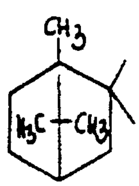 2-α(cis, trans-Gemisch) | O | Cl | Cl | H | 172-174 |

Entsprechend den Beispielen werden die Verbindungen der Formel I der nachstehenden Tabelle erhalten:

Tabelle I

| Nr. | A | W | X | Y | Fp. [°C] |
|---|---|---|---|---|---|
| 1 | 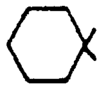 | O | $CH_3$ | $CH_3$ | 224-226 |
| 2 | 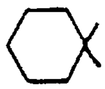 | O | H | $CH_3$ | 198-199 |
| 3 | 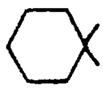 | O | H | $CF_3$ | 222-223 |
| 4 | 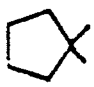 | O | Cl | Cl | 134-135 |
| 5 | 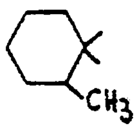 (2-cis) | O | Cl | Cl | 205-206 |
| 6 | 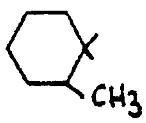 (2-cis) | O | $CH_3$ | $CH_3$ | 202-203 |
| 7 | 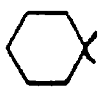 | O | Cl | Cl | 258-263 |
| 8 | 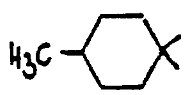 (4-cis) | O | Cl | Cl | 253-255 |
| 9 | 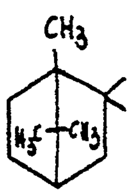 2-d-(cis, trans-Gemisch) | O | Cl | Cl | 207-208 |
| 10 | 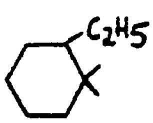 (cis/trans) | O | Cl | Cl | |
| 11 | 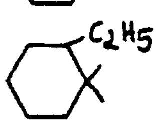 (cis/trans) | O | $CH_3$ | Cl | |

| Nr. | A | | W | X | Y | Fp. [°C] |
|---|---|---|---|---|---|---|
| 12 | 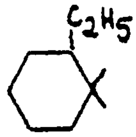 | (cis) | O | Br | Cl | |
| 13 | 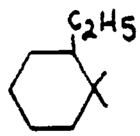 | (cis) | O | $CF_3$ | $CF_3$ | |
| 14 | 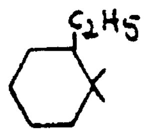 | (cis) | O | $OCH_3$ | $OCH_3$ | |
| 15 | 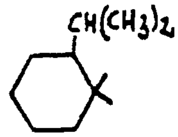 | (cis/trans) | O | Cl | Cl | |
| 16 | 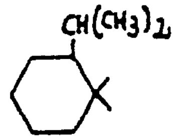 | (trans) | O | $CH_3$ | $OCH_3$ | |

| Nr. | A | | W | X | Y | Fp. [°C] |
|---|---|---|---|---|---|---|
| 17 | 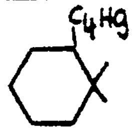 | (cis) | O | Cl | Cl | |
| 18 | 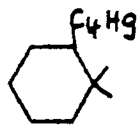 | (trans) | O | $CF_3$ | $CF_3$ | |
| 19 | 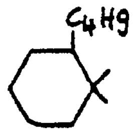 | (cis/trans) | O | $CH_3$ | $CH_3$ | |
| 20 | 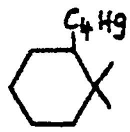 | (cis/trans) | O | $OCH_3$ | $OCH_3$ | |
| 21 | 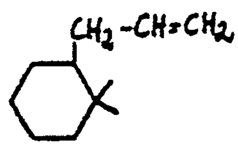 | (cis) | O | Cl | Cl | |
| 22 | 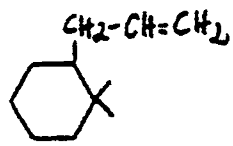 | (cis) | O | $CH_3$ | Cl | |
| 23 | 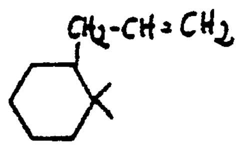 | (trans) | O | $CF_3$ | Cl | |

| Nr. | A | W | X | Y | Fp. [°C] |
|---|---|---|---|---|---|
| 24 | 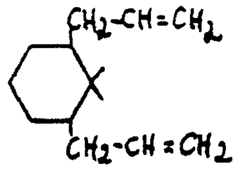 (2-trans) | O | Cl | Cl | |
| 25 | 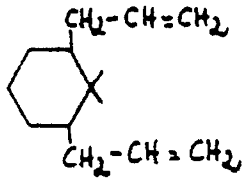 (2-trans) | O | Br | Br | |
| 26 | 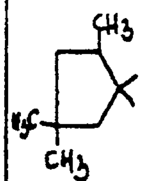 (trans) | O | Cl | Cl | |
| 27 | 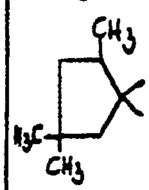 (cis) | O | Cl | Cl | |
| 28 | 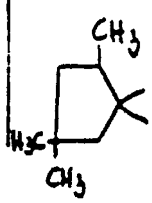 (trans) | O | $CH_3$ | Cl | |

| Nr. | A | W | X | Y | Fp. [°C] |
|---|---|---|---|---|---|
| 29 | 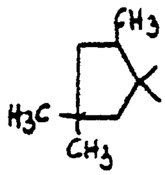 (cis) | O | $OCH_3$ | $OCH_3$ | |
| 30 | 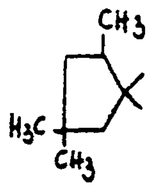 (trans) | O | $CF_3$ | $CF_3$ | |
| 31 | 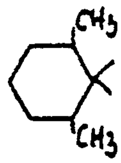 (trans) | O | Br | Br | |
| 32 | 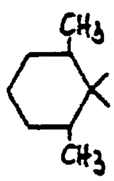 (cis) | O | $CH_3$ | $CH_3$ | |
| 33 | 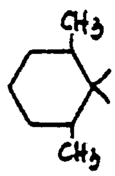 (cis) | O | $CF_3$ | $CF_3$ | |
| 34 | 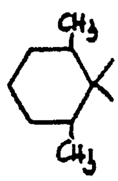 (trans) | O | $OCH_3$ | $OCH_3$ | |

Beispiel 2

cis-4-Methylcyclohexan-5'-spiro-3'-(3,5-dibromphenyl) hydantoin

a) cis-4-Methylcyclohexan-5'-spiro-3'-(2-nitrophenyl)-hydantoin
18,2 g (0,1 mol) cis-4-Methylcyclohexan-5'-spiro-hydantoin, 18 g (0,1 mol) 2-Nitro-chlorbenzol und 41,4 g (0,3 mol) Kaliumcarbonat werden in 100 ml Dimethylformamid gelöst bzw. suspendiert und 5 Stunden bei ca. 120 °C gerührt.
Man gießt die Mischung anschließend auf Eis, saugt den ausgefallenen Niederschlag ab, wäscht gründlich mit Wasser und kristallisiert aus Acetonitril.
Ausbeute: 15,5 g (51 % d.Th.)
Fp.: 227 °C - 229 °C.
b) cis-4-Methylcyclohexan-5'-spiro-3'-(2-aminophenyl)-hydantoin
15,2 g (0,05 mol) cis-4-Methylcyclohexan-5'-spiro-3'-(2-nitrophenyl)-hydantoin werden in 200 ml Methylalkohol und 5 ml konz. Salzsäure gelöst und nach Zugabe von 4 g Katalysator (Pd/C 10 %ig) bei max. 40 °C und einem Druck von 5 bar hydriert. Nach beendeter $H_2$-Aufnahme wird der Katalysator abgesaugt und die Mutterlauge eingeengt. Man nimmt den Rückstand in Wasser auf und neutralisiert mit Bicarbonat. Der entstandene Feststoff wird abgesaugt, mit Wasser gewaschen und aus Isopropanol kristallisiert.
Ausbeute: 11,5 g (84 % d.Th.)
Fp. 255 °C - 256 °C.

c) cis-4-Methylcyclohexan-5'-spiro-3'-(2-amino-3,5-dibromphenyl)-hydantoin

27,33 g (0,1 mol) cis-4-Methyl-cyclohexan-5'-spiro-3'-(2-amino-phenyl)-hydantoin werden in 200 ml Eisessig gelöst. Dazu tropft man bei Raumtemperatur eine Lösung von 32 g (0,2 mol) Brom in 50 ml Eisessig innerhalb von 30 Minuten ein. Man rührt ca. 30 Minuten nach, verdünnt dann mit 1 Ltr. Wasser, saugt den Niederschlag ab, wäscht mit Wasser und kristallisiert aus Acetonitril.
Ausbeute: 35,4 g (82 % d.Th.)
Fp. 264 - 266 °C

d) cis-4-Methylcyclohexan-5'-spiro-3'-(3,5-dibromphenyl)-hydantoin

21,6 g (0,05 mol) cis-4-Methyl-5'-spiro-3'-(2-amino-3,5-dibromphenyl)-hydantoin werden in 300 ml Ethanol und 10 ml konz. Schwefelsäure gelöst, und die Mischung wird zum Sieden erhitzt. Dazu gibt man innerhalb von ca. 20 Minuten portionsweise 3,8 g (0,055 mol) Natriumnitrit und rührt dann eine weitere Stunde unter Rückfluß. Nach dem Abkühlen wird mit 1 Ltr. Eiswasser verdünnt, der entstandene Niederschlag abgesaugt, mit Wasser gewaschen und aus Acetonitril kristallisiert.
Ausbeute: 12,3 g ( 59 % d.Th.)
Fp. 247-249 °C.

## Beispiel 3

cis-2-Ethylcyclohexan-5'-spiro-3'-(3,5-dibromphenyl)-hydantoin

a) cis-2-Ethylcyclohexan-5'-spiro-3'-(2,4-dinitrophenyl)-hydantoin

39 g (0,2 mol) cis-2-Ethylcyclohexan-5'-spiro-hydantoin, 40,4 g (0,2 mol) 1-Chlor-2,4-dinitrobenzol, 83 g (0,6 mol) Kaliumcarbonat und 1 g Tetrabutylammonium-hydrogensulfat werden in 200 ml Dimethylsulfoxid bei 10 °C 6 Stunden gerührt. Danach verdünnt man mit 500 ml Wasser, saugt ab, wäscht mit Wasser und kaltem Methanol und trocknet.
Ausbeute: 62,4 g (86 %d.Th.)
Fp. 231 - 232 °C

b) cis-2-Ethylcyclohexan-5'-spiro-3'-(2,4-diaminophenyl)-hydantoin

36,2 g (0,1 mol) cis-2-Ethylcyclohexan-5'-spiro-3'-(2,4-dinitrophenyl)-hydantoin werden in 500 ml Methanol und 20 ml konz. Salzsäure gelöst. Dazu gibt man 10 g Katalysator (Pd/C 10 %ig) und hydriert unter 5 bar bei einer Temperatur von max. 65 °C. Danach wird vom Katalysator abgesaugt, eingeengt und in Wasser aufgenommen. Man neutralisiert mit Bicarbonat, saugt den ausgefallenen Niederschlag ab, wäscht mit Wasser und kristallisiert aus Isopropanol.
Ausbeute: 25,4 g (84 % d.Th.)
Fp. 183 - 185 °C.

c) cis-2-Ethylcyclohexan-5'-spiro-3'-(2,4-diamino-3,5-dibromphenyl)-hydantoin

15 g (0,05 mol) cis-2-Ethylcyclohexan-5'-spiro-3'-(2,4-diaminophenyl)-hydantoin werden in 100 ml Eisessig gelöst. Dazu tropft man bei Raumtemperatur eine Lösung von 5,06 g (0,1 mol) Brom in 20 ml Eisessig innerhalb von 10 Minuten ein. Man rührt ca. 20 Minuten nach, versetzt dann mit 300 ml Eiswasser, saugt den Feststoff ab, wäscht mit Wasser gründlich nach und trocknet im Vakuum.
Ausbeute: 18 g (78,3 % d.Th.)
Fp. 263 - 265 °C.

d) cis-2-Ethylcyclohexan-5'-spiro-3'-(3,5-dibromphenyl)-hydantoin

Man erwärmt eine Lösung von 3,5 g (0,03 mol) Isoamylnitrit in 50 ml Dimethylformamid auf 55 °C und tropft dazu bei dieser Temperatur eine Lösung von 6,9 g (0,015 mol) cis-2-Ethyl-cyclohexan-5'-spiro-3'-(2,4-diamino-3,5-dibrom-phenyl)-hydantoin in 20 ml Dimethylformamid innerhalb einer Stunde ein. Man erhöht anschließend die Temperatur bis zum Ende der $N_2$-Entwicklung auf 70 - 80 °C. Dann wird im Vakuum eingeengt und der dunkle Rückstand säulenchromatographisch (Kieselgel/Essigester) gereinigt.
Ausbeute: 3,9 g (59 % d.Th.)
Fp. 213 - 215 °C.

Entsprechend erhält man die Verbindungen der Tabelle II.

Tabelle II

| Nr. | A | W | X | Y | Fp °C |
|---|---|---|---|---|---|
| 1 | 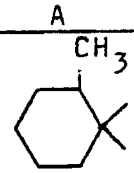 (cis) | O | Br | $CH_3$ | 193-195 |
| 2 | 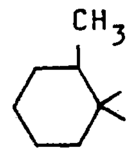 (cis) | O | Cl | $CH_3$ | 202-203 |
| 3 | 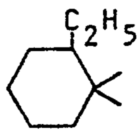 (cis) | O | Cl | Cl | 193-195 |
| 4 | 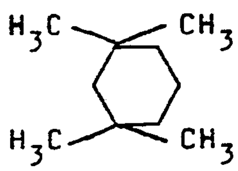 | O | Cl | Cl | 212-213 |
| 5 | 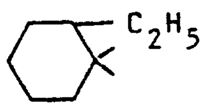 (cis) | O | $CH_3$ | $CH_3$ | 162-163 |
| 6 | 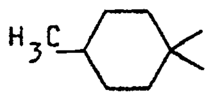 | O | $CH_3$ | $CH_3$ | 208-211 |
| 7 | 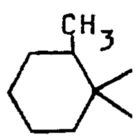 (trans) | O | Cl | Cl | 188-189 |
| 8 | 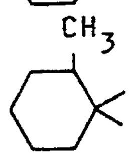 (cis) | O | $OCH_3$ | $OCH_3$ | 177-179 |
| 9 | 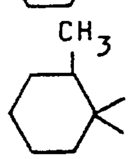 (cis) | O | $CF_3$ | $CF_3$ | 179-181 |

Beispiel 4

cis-2-Methylcyclohexan-5'-spiro-3'-(2,6-dichlorpyridyl-4)-hydantoin

192 g (1 mol) cis-2-Methylcyclohexan-5'- spiro-hydantoin, 194 g (1 mol) 2,6-Dichlor-4-nitropyridin, 207 g

(1,5 mol) Kaliumcarbonat und 3,4 g (0,01 mol) Tetrabutylammonium-hydrogensulfat werden in 1000 ml Dimethylsulfoxid bei Raumtemperatur gelöst bzw. suspendiert. Danach wird das Gemisch weitere ca. 16 Stunden bei 15° C - 20° C gerührt.
Man läßt nun ca. 5000 ml Eiswasser zulaufen, saugt den gebildeten Niederschlag ab, wäscht gründlich mit Wasser nach und kristallisiert aus Acetonitril um.
Ausbeute: 250 g (79 %d.Th.)
Fp. 227 - 229° C.

Entsprechend werden die Verbindungen der Tabelle III erhalten.

Tabelle III

| Nr. | A | X | Y | Fp °C |
|---|---|---|---|---|
| 1 | 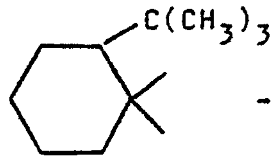 - cis - | Cl | Cl | 216-218 |
| 2 | 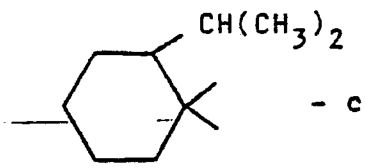 - cis - | Cl | Cl | 190-191 |
| 3 | 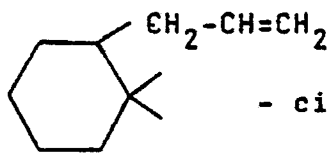 - cis - | Cl | Cl | 138-140 |
| 4 | 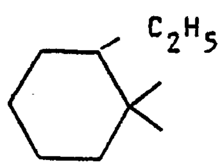 - cis - | Cl | Cl | 191-193 |
| 5 | 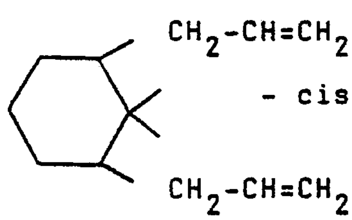 - cis - | Cl | Cl | 125-127 |
| 6 | 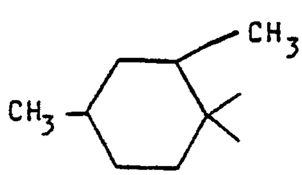 Isomerengemisch | Cl | Cl | 238-242 |

Beispiel 5

5-Methyl-5-cyclopropyl-3-[4 -(2 ,6 -dichlorpyridyl)]-hydantoin

Eine Suspension, bestehend aus 3,1 g 5-Methyl-5-cyclopropyl-hydantoin, 3,86 g 2,6-Dichlor-4-nitropyridin und 5,6 g Kaliumcarbonat in 10 ml Dimethylformamid, wird 18 Stunden bei 20° C gerührt und anschließend auf 100 ml Wasser gegeben. Nach 30 Minuten Rühren wird der entstandene Niederschlag abgesaugt und in Methylenchlorid aufgenommen. Nach dem Trocknen und Abziehen des Lösungsmittels erhält man 3,2 g 5-Methyl-5-cyclopropyl-3-[4 -(2 ,6 -dichlorpyridyl)]-hydantoin, Fp. 160° C (Umfällung aus Toluol/Petrolether).
Ausbeute 64% d.Th.

Beispiel 6

5-Methyl-5-cyclopropyl-3-(3 ,5 -dimethylphenyl)-hydantoin Zu einer Lösung von 0,8 g 1-(3,5-Dimethylphenyl)-3-[2-(2-cyclopropyl)-propansäuremethylester]-harnstoff in 30 ml Methanol gibt man 0,3 g Triethylamin und erhitzt 12 Stunden zum Rückfluß. Nach dem Abziehen des Lösungsmittels wird der Rückstand mit Petrolether verrieben und der erhaltene kristalline Niederschlag abgesaugt.
Fp. 121° C, Ausbeute 0,6 g (85 % d.Th.).

Analog den Beispielen erhält man die Verbindungen der Tabelle IV.

Tabelle IV

Endprodukte der Formel I (A gleich 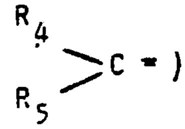 )

| Nr. | Q | W | X | Y | $R_4$ | $R_5$ | Fp[°C] |
|---|---|---|---|---|---|---|---|
| 1 | N | O | Cl | Cl | $CH_3$ | $i-C_3H_7$ | 129 |
| 2 | N | O | Cl | Cl | $i-C_3H_7$ | $i-C_3H_7$ | 157-159 |
| 3 | N | O | Cl | Cl | $CH_3$ | $t-C_4H_9$ | 167-170 |
| 4 | N | O | Cl | Cl | $n-C_3H_7$ | $i-C_3H_7$ | 91-93 |
| 5 | N | O | Cl | Cl | $CH_3$ | $-CH_2-CH_2-CH=CH_2$ | 76-81 |
| 6 | N | O | Cl | Cl | $C_2H_5$ | $i-C_3H_7$ | 97-103 |
| 7 | N | O | Cl | Cl | $C_6H_5$ | $C_6H_5$ | 190-194 |
| 8 | N | O | Cl | Cl | $CH_3$ | $C_6H_5$ | 167-170 |
| 9 | N | O | Cl | Cl | $CH_3$ | $n-C_4H_9$ | |
| 10 | N | O | Cl | Cl | $CH_3$ | 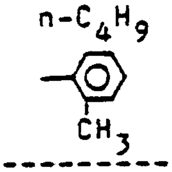 | 161-163 |
| 11 | N | O | Cl | CL | $CH_3$ | $i-C_4H_9$ | 133-138 |
| 12 | N | O | Cl | Cl | $CH_3$ | $s-C_4H_9$ | 105-112 |
| 13 | N | O | Cl | Cl | $C_2H_5$ | $t-C_4H_9$ | |
| 14 | CH | O | Cl | Cl | $CH_3$ | $i-C_3H_7$ | 124 |
| 15 | CH | O | $CH_3$ | $CH_3$ | $CH_3$ | $i-C_3H_7$ | 134-136 |
| 16 | CH | O | Cl | Cl | $CH_3$ | 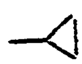 | 145 |
| 17 | CH | O | Cl | Cl | $CH_3$ | $t-C_4H_9$ | 230 |

| Nr. | Q | W | X | Y | $R_4$ | $R_5$ | Fp[°C] |
|---|---|---|---|---|---|---|---|
| 18 | CH | O | $CH_3$ | $CH_3$ | $CH_3$ | $t-C_4H_9$ | 153 |
| 19 | CH | O | Cl | Cl | $n-C_3H_7$ | $i-C_3H_7$ | 140-145 |
| 20 | CH | O | $CH_3$ | $CH_3$ | $n-C_3H_7$ | $i-C_3H_7$ | 110 |
| 21 | CH | O | Cl | Cl | $CH_3$ | $-CH_2-CH_2-CH=CH_2$ | 80 |
| 22 | CH | O | Cl | Cl | $CH_3$ | $n-C_4H_9$ | 100 |
| 23 | CH | O | $CH_3$ | $CH_3$ | $CH_3$ | $n-C_4H_9$ | 122 |
| 24 | CH | O | $CH_3$ | $CH_3$ | $CH_3$ | $-CH_2-CH_2-CH=CH_2$ | 90 |
| 25 | CH | O | Cl | Cl | $CH_3$ | $C_6H_5$ | 158 |
| 26 | CH | O | $CH_3$ | $CH_3$ | $CH_3$ | $C_6H_5$ | 168 |
| 27 | CH | O | Cl | CL | $C_6H_5$ | $C_6H_5$ | 136 |
| 28 | CH | O | $CH_3$ | $CH_3$ | $C_6H_5$ | $C_6H_5$ | |
| 29 | CH | O | Cl | Cl | $CH_3$ | 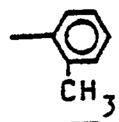 | 205 |
| 30 | CH | O | $CH_3$ | $CH_3$ | $CH_3$ | 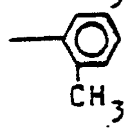 | |
| 31 | CH | O | Cl | Cl | $i-C_3H_7$ | $i-C_3H_7$ | 210 |
| 32 | CH | O | $CH_3$ | $CH_3$ | $i-C_3H_7$ | $i-C_3H_7$ | |

| Nr. | Q | W | X | Y | $R_4$ | $R_5$ | Fp [°C] |
|---|---|---|---|---|---|---|---|
| 33 | CH | O | Cl | Cl | $CH_3$ | $i-C_4H_9$ | |
| 34 | CH | O | $CH_3$ | $CH_3$ | $CH_3$ | $i-C_4H_9$ | |
| 35 | CH | O | Cl | Cl | $C_2H_5$ | $i-C_3H_7$ | |
| 36 | CH | O | $CH_3$ | $CH_3$ | $C_2H_5$ | $i-C_3H_7$ | |
| 37 | CH | O | Cl | Cl | $CH_3$ | $s-C_4H_9$ | 130 |
| 38 | CH | O | $CH_3$ | $CH_3$ | $CH_3$ | $s-C_4H_9$ | |
| 39 | CH | O | Cl | Cl | $C_2H_5$ | $t-C_4H_9$ | |
| 40 | CH | O | $CF_3$ | $CF_3$ | $CH_3$ | $i-C_3H_7$ | 124 |
| 41 | CH | O | $OCH_3$ | $OCH_3$ | $CH_3$ | $i-C_3H_7$ | 129-130 |
| 42 | CH | O | Br | Br | $CH_3$ | $i-C_3H_7$ | 140 |
| 43 | CH | O | Cl | $CH_3$ | $CH_3$ | $i-C_3H_7$ | 120 |
| 44 | CH | O | Br | $CH_3$ | $CH_3$ | $i-C_3H_7$ | 135 |
| 45 | CH | O | Cl | Br | $C_2H_5$ | 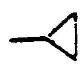 | |
| 46 | N | O | $OCH_3$ | Cl | $i-C_3H_7$ | $i-C_3H_7$ | |
| 47 | CH | O | $CF_3$ | Cl | $CH_3$ | 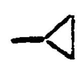 | |

| Nr. | Q | W | X | Y | $R_4$ | $R_5$ | Fp [°C] |
|---|---|---|---|---|---|---|---|
| 48 | CH | O | Cl | Cl | $CH_3$ | $CH_2-O-CH_3$ | 134 |
| 49 | CH | O | Cl | Cl | $CH_3$ | 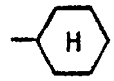 | 185 |
| 50 | CH | O | Cl | Cl | $C_2H_5$ | $i-C_4H_9$ | 140 |
| 51 | CH | O | Cl | Cl | $CH_3$ | $CH_2-C_6H_5$ | 142 |
| 52 | N | O | Cl | Cl | $C_6H_5$ | 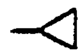 | 137-140 |
| 53 | N | O | Cl | Cl | 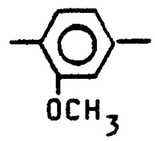 | $CH_3$ | 228-233 |
| 54 | N | O | Cl | Cl | $CH_3$ | $-CH-O-CH_3$ | 156-158 |
| 55 | N | O | Cl | Cl | $CH_3$ | 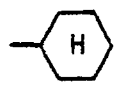 | 148-155 |
| 56 | N | O | Cl | Cl | 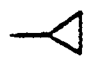 | 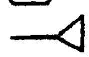 | 195-199 |
| 57 | N | O | Cl | Cl | $CH_3$ | $-CH_2-O-C_6H_5$ | 128-135 |

Ausgangs- bzw. Zwischenprodukte zu Tabelle IV

Tabelle V

Hydantoine der Formel

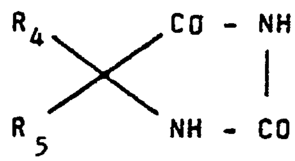

| Nr. | $R_4$ | $R_5$ | Fp [°C] |
|---|---|---|---|
| 1 | $i\text{-}C_3H_7$ | $CH_3$ | 176 - 177 |
| 2 | $t\text{-}C_4H_9$ | $CH_3$ | 220 |
| 3 | 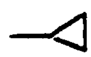 | $CH_3$ | 149 - 150 |
| 4 | $CH_2{=}CH\text{-}CH_2\text{-}CH_2$ | $CH_3$ | 117 |
| 5 | $n\text{-}C_3H_7$ | $i\text{-}C_3H_7$ | 188 |
| 6 | $i\text{-}C_3H_7$ | $i\text{-}C_3H_7$ | 210 - 212 |
| 7 | $n\text{-}C_4H_9$ | $CH_3$ | 105 - 106 |
| 8 | $C_6H_5$ | $CH_3$ | 200 |
| 9 | $C_6H_5$ | $C_6H_5$ | 300 |
| 10 | $C_3H_7$ | $CH_3$ | 124-126 |
| 11 | $i\text{-}C_4H_9$ | $CH_3$ | 147 |
| 12 | $i\text{-}C_3H_7$ | $C_2H_5$ | |
| 13 | $t\text{-}C_4H_9$ | $C_2H_5$ | |
| 14 | $s\text{-}C_4H_9$ | $CH_3$ | 179-189 |
| 15 | 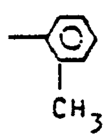 | $CH_3$ | 158 |

| Nr. | $R_4$ | $R_5$ | Fp [°C] |
|---|---|---|---|
| 16 | 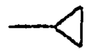 | 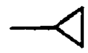 | 199-200 |
| 17 | $CH_2-O-CH_3$ | $CH_3$ | 170-173 |
| 18 | 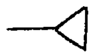 | $-C_6H_5$ | 214 |
| 19 | $CH_3$ | 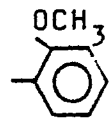 | 226-228 |
| 20 | $C_6H_5$ | 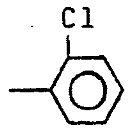 | > 250 |

Tabelle VI

Aminocarbonsäure -hydrochloride der Formel

$$HOOC - \underset{R_3}{\overset{R_4}{C}} - NH_2 \cdot HCl$$

| Nr. | $R_4$ | $R_5$ | Fp. [°C] |
|---|---|---|---|
| 1 | $t-C_4H_9$ | $CH_3$ | 250 |
| 2 | $i-C_3H_7$ | $CH_3$ | 250 |
| 3 | 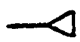 | $CH_3$ | ca.255 |
| 4 | $CH_2=CH-CH_2-CH_2$ | $CH_3$ | 250 |
| 5 | $CH_3-CH_2-CH_2$ | $i-C_3H_7$ | ca.230 |
| 6 | $i-C_3H_7$ | $i-C_3H_7$ | |
| 7 | $n-C_4H_9$ | $CH_3$ | 230 |
| 8 | $C_6H_5$ | $CH_3$ | > 250 |
| 9 | $C_6H_5$ | $C_6H_5$ | >250 |
| 10 | 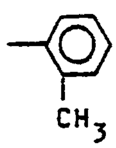 | $CH_3$ | amorph |
| 11 | $C_3H_7$ | $CH_3$ | 215 |
| 12 | $i-C_4H_9$ | $CH_3$ | |
| 13 | $i-C_3H_7$ | $C_2H_5$ | |
| 14 | $t-C_4H_9$ | $C_2H_5$ | |
| 15 | $s-C_4H_9$ | $CH_3$ | |
| 16 | 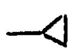 | $C_6H_5$ | 240 |
| 17 | $CH_3$ | 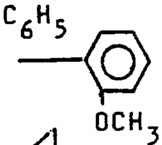 | >220 |
| 18 | 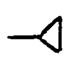 | 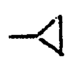 | >250 |

Tabelle VII

Aminocarbonsäurederivate 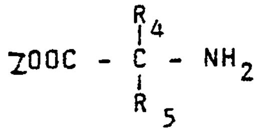

| Nr. | $R_4$ | $R_5$ | Z | Physikalische Daten Fp. [°C] |
|---|---|---|---|---|
| 1 | $i-C_3H_7$ | $CH_3$ | $CH_3$ | Kp.69-70°C/ 21 mbar |
| 2 | $t-C_4H_9$ | $CH_3$ | $CH_3$ | |
| 3 | | $CH_3$ | $CH_3$ | |
| 4 | $CH_2=CH-CH_2-CH_2$ | $CH_3$ | $CH_3$ | |
| 5 | $n-C_3H_7$ | $i-C_3H_7$ | $CH_3$ | |
| 6 | $n-C_4H_9$ | $CH_3$ | $C_2H_5$ | |
| 7 | $C_6H_5$ | $CH_3$ | $CH_3$ | |
| 8 | $C_6H_5$ | $C_6H_5$ | $CH_3$ | |
| 9 | 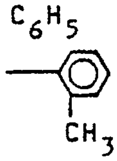 | $CH_3$ | $CH_3$ | |
| 10 | $i-C_3H_7$ | $CH_3$ | $C_2H_5$ | |
| 11 | $i-C_3H_7$ | $CH_3$ | $i-C_3H_7$ | |
| 12 | $i-C_3H_7$ | $CH_3$ | $CH_2=CH-CH_2$ | |
| 13 | $i-C_3H_7$ | $CH_3$ | $C_6H_5$ | |
| 14 | $C_2H_5$ | $CH_3$ | $CH_3$ | |
| 15 | $i-C_4H_9$ | $CH_3$ | $CH_3$ | |
| 16 | $i-C_3H_7$ | $C_2H_5$ | $CH_3$ | |
| 17 | $s-C_4H_9$ | $CH_3$ | $CH_3$ | |
| 18 | $t-C_4H_9$ | $C_2H_5$ | $CH_3$ | |
| 19 | $CH_3$ | $i-C_3H_7$ | $n-C_4H_8-OCH_3$ | |
| 20 | $CH_3$ | $i-C_3H_7$ | $C_2H_4-OC_2H_5$ | |

Die Verbindungen der vorstehenden Tabelle wurden durch MNR- und IR-Spektrum charakterisiert.

Tabelle VIII

Verbindungen der Formel 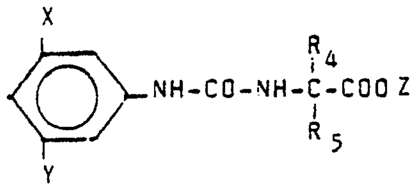

| Nr. | X | Y | $R_4$ | $R_5$ | Z | Fp[°C] |
|---|---|---|---|---|---|---|
| 1 | Cl | Cl | $CH_3$ | $i-C_3H_7$ | $CH_3$ | 178 |
| 2 | Cl | Cl | $CH_3$ | 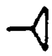 | $CH_3$ | 168 |
| 3 | Cl | Cl | $CH_3$ | $t-C_4H_9$ | $CH_3$ | 194 |
| 4 | Cl | Cl | $n-C_3H_7$ | $i-C_3H_7$ | $CH_3$ | 145 |
| 5 | Cl | Cl | $CH_3$ | $CH_2{=}CH-CH_2-CH_2$ | H | amorph |
| 6 | $CH_3$ | $CH_3$ | $CH_3$ | $i-C_3H_7$ | $CH_3$ | 182 |
| 7 | $CH_3$ | $CH_3$ | $CH_3$ | 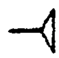 | $CH_3$ | 161 |
| 8 | $CH_3$ | $CH_3$ | $CH_3$ | $t-C_4H_9$ | $CH_3$ | 206 |
| 9 | Cl | Cl | $CH_3$ | $i-C_3H_7$ | $C_2H_5$ | 155-158 |
| 10 | Cl | Cl | $CH_3$ | $i-C_3H_7$ | $i-C_3H_7$ | 85-90 |
| 11 | Cl | Cl | $CH_3$ | $i-C_3H_7$ | $CH_2{=}CH-CH_2$ | 144 |
| 12 | Cl | Cl | $CH_3$ | $i-C_3H_7$ | $C_6H_5$ | |
| 13 | Cl | Cl | $CH_3$ | $n-C_4H_9$ | $C_2H_5$ | 138-140 |
| 14 | $CH_3$ | $CH_3$ | $CH_3$ | $n-C_4H_9$ | $C_2H_5$ | 134 |
| 15 | $CH_3$ | $CH_3$ | $CH_3$ | $CH_2{=}CH-CH_2-CH_2$ | H | |
| 16 | Cl | Cl | $CH_3$ | $C_6H_5$ | $C_2H_5$ | 180 |
| 17 | $CH_3$ | $CH_3$ | $CH_3$ | $C_6H_5$ | $C_2H_5$ | 185 |
| 18 | Cl | Cl | $C_6H_5$ | $C_6H_5$ | $C_2H_5$ | 200 |
| 19 | $CH_3$ | $CH_3$ | $C_6H_5$ | $C_6H_5$ | $CH_3$ | |
| 20 | Cl | Cl | $CH_3$ | 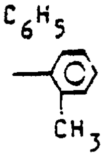 | $C_2H_5$ | 223 |
| 21 | $CH_3$ | $CH_3$ | $CH_3$ | 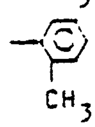 | H | 205 |

| Nr. | X | Y | $R_4$ | $R_5$ | Z | Fp[°C] |
|---|---|---|---|---|---|---|
| 22. | Cl | Cl | $i-C_3H_7$ | $i-C_3H_7$ | H | 75-80 |
| 23 | $CH_3$ | $CH_3$ | $i-C_3H_7$ | $i-C_3H_7$ | H | |
| 24 | Cl | Cl | $CH_3$ | $i-C_4H_9$ | $CH_3$ | |
| 25 | $CH_3$ | $CH_3$ | $CH_3$ | $i-C_4H_9$ | $CH_3$ | |
| 26 | Cl | Cl | $C_2H_5$ | $i-C_3H_7$ | $CH_3$ | 185 |
| 27 | $CH_3$ | $CH_3$ | $C_2H_5$ | $i-C_3H_7$ | $CH_3$ | |
| 28 | Cl | Cl | $CH_3$ | $s-C_4H_9$ | $CH_3$ | 174-176 |
| 29 | $CH_3$ | $CH_3$ | $CH_3$ | $s-C_4H_9$ | $CH_3$ | |
| 30 | Cl | Cl | $C_2H_5$ | $t-C_4H_9$ | $CH_3$ | |
| 31 | $CF_3$ | $CF_3$ | $CH_3$ | $i-C_3H_7$ | $C_2H_5$ | 160 |
| 32 | $OCH_3$ | $OCH_3$ | $CH_3$ | $i-C_3H_7$ | $C_2H_5$ | 160 |
| 33 | Br | Br | $CH_3$ | $i-C_3H_7$ | $C_2H_5$ | 190 |
| 34 | $CH_3$ | Cl | $CH_3$ | 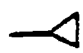 | $C_2H_4-OCH_3$ | |
| 35 | $CH_3$ | Cl | $CH_3$ | $i-C_3H_7$ | $n-C_4H_9$ | |
| 36 | Cl | $OCH_3$ | $CH_3$ | $i-C_3H_7$ | $n-C_4H_8-OCH_3$ | |
| 37 | Cl | $CF_3$ | $CH_3$ | $i-C_3H_7$ | $C_2H_4-O-C_2H_5$ | |
| 38 | Cl | $CH_3$ | $CH_3$ | $i-C_3H_7$ | $C_2H_5$ | 165 |
| 39 | Br | $CH_3$ | $CH_3$ | $i-C_3H_7$ | $C_2H_5$ | 172 |
| 40 | $CH_3$ | $CH_3$ | $i-C_3H_7$ | $n-C_4H_9$ | $CH_3$ | 123 |
| 41 | Cl | Cl | $CH_3$ | $CH_2-O-CH_3$ | $CH_3$ | 145-148 |
| 42 | Cl | Cl | $CH_3$ | 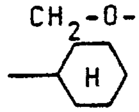 | $CH_3$ | 200-205 |
| 43 | $CH_3$ | $CH_3$ | $CH_3$ | $CH_2-CH_2-CH=CH_2$ | $C_2H_5$ | 130 |
| 44 | Cl | Cl | $CH_3$ | $CH_2-CH_2-CH=CH_2$ | $C_2H_5$ | 140 |

| Nr. | X | Y | $R_4$ | $R_5$ | Z | Fp °C |
|---|---|---|---|---|---|---|
| 45 | Cl | Cl | $C_2H_5$ | $t-C_4H_9$ | $C_2H_5$ | 140 |
| 46 | Cl | Cl | $CH_3$ | $-CH_2-C_6H_5$ | $C_2H_5$ | 180 |
| 47 | Cl | Cl | $CH_3$ | 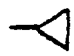 | H | 167 |
| 48 | $CH_3$ | $CH_3$ | $CH_3$ | 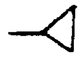 | H | 159 |
| 49 | $CH_3$ | $CH_3$ | $CH_3$ | $i-C_3H_7$ | H | 185 |
| 50 | Cl | Cl | $CH_3$ | $i-C_3H_7$ | H | 188 |
| 51 | $CH_3$ | $CH_3$ | $CH_3$ | $i-C_3H_7$ | $C_2H_5$ | 145-147 |

Wirkung und Verträglichkeit der erfindungsgemäßen Verbindungen wurden in Gewächshausversuchen geprüft. Bei der Bonitierung wurde ein zehnstufiger Bonitierungsschlüssel angewendet, wobei 1 100 % Wirkung, 10 keine Wirkung bedeutet. Die Pflanzen I bis III sind Unkräuter, IV bis VI Nutzpflanzen.

| Wirkstoff nach | Aufwand-menge kg/ha | I | II | III | IV | V | VI |
|---|---|---|---|---|---|---|---|
| Beisp. 1 | 1 | 1 | 1 | 1 | 10 | 10 | 10 |
| | 0,5 | 2 | 1 | 1 | 10 | 10 | 10 |
| Tab. I Nr. 5 | 1 | 1 | 1 | 1 | - | 10 | 10 |
| | 0,5 | 1 | 1 | 1 | - | 10 | 10 |

I: Echinocloa crus-galli
II: Cynodon dactylon
III: Digitaria sanguinalis
IV: Oryza sativa
V: Gossypium hirsutum
VI: Glyzine max.

Wie die Tabelle zeigt, verbinden die erfindungsgemäßen Wirkstoffe sehr gute Wirkung (weit überwiegend Bonitierungsnote 1) mit ausgezeichneter Verträglichkeit (in allen Fällen Bonitierungsnote 10).

**Patentansprüche**

1. Verbindungen der Formel

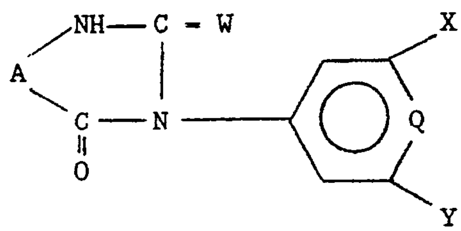

(I),

in der

A für einen gegebenenfalls ein-oder mehrfach verbrückten Cycloalkanrest der Formel

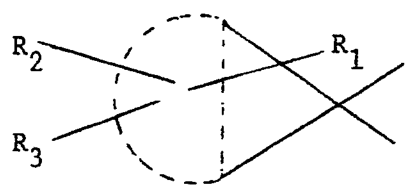

(II)

mit 5 bis 10 C-Atomen oder für den Rest = $CR_4R_5$,

Q für CH oder N,

$R_1$, $R_2$ und $R_3$, die gleich oder verschieden sein Können, für Wasserstoff, für geradkettiges oder verzweigtes $C_1$-$C_4$-Alkyl oder für geradkettiges oder verzweigtes $C_3$-$C_4$-Alkenyl,

$R_4$ und $R_5$, die gleich oder verschieden sein können, für $C_1$-$C_4$-Alkyl, das auch durch $C_1$-$C_4$-Alkyl-O-oder $C_1$-$C_4$-Alkyl-S- oder eine gegenbenenfalls ein- oder mehrfach durch $C_1$-$C_4$-Alkyl, $C_1$-$C_4$-Alkoxy, Trifluoromethyl und Halogen substituierte Phenyl-O- oder Phenyl-S-Gruppe substituiert sein kann, für $C_2$-$C_4$-Alkenyl, für $C_3$-$C_6$-Cycloalkyl, das auch niederalkylsubstituiert sein kann, für gegebenenfalls ein- oder mehrfach durch $C_1$-$C_4$-Alkyl, $C_1$-$C_4$-Alkoxy, Trifluoromethyl und Halogen substituierte Phenyl oder Benzyl, mit der Massgabe, dass wenn $R_4$ nichtsubstituiertes Methyl ist, $R_5$ nicht für Methyl oder Ethyl steht,

W für Sauerstoff, und

X und Y, die gleich oder verschieden sein können, für Halogen, $C_1$-$C_4$-Alkyl, $C_1$-$C_4$-Alkoxy oder Trifluormethyl, X ausserdem für Wasserstoff

steht, mit der Massgabe, dass wenn A eine nichtsubstituierte Cycloalkylgruppe und Q CH ist, X nicht für Wasserstoff stehen kann.

2. Verbindungen nach Anspruch 1, wobei X für Wasserstoff, Chlor, Brom, Methyl, Methoxy oder Trifluormethyl, Y für Chlor, Brom, Methyl, Methoxy oder Trifluormethyl steht.

3. Herbizide Mittel, gekennzeichnet durch einen Gehalt an einer Verbindung nach Anspruch 1 oder 2, neben üblichen Hilfs- und/oder Trägerstoffen.

4. Herbizide Mittel, dadurch gekennzeichnet, dass sie neben einem Wirkstoff nach Anspruch 1 oder 2 einen weiteren herbiziden Wirkstoff aus der Gruppe der harnstoff-Derivate, Triazin-Derivate, Dinitroanilin-Derivative, Chloracetanilid-Derivate oder der Thiocarbamate enthält.

5. Verwendung von Verbindungen der Formel I bei der Bekämpfung von Unkräutern und Ungräsern in Nutzpflanzen-Kulturen, insbesondere in Soja, Mais, Reis, Baumwolle, Gerste, Rüben, Kartoffeln, Hirse, Tomaten, Zwiebeln.

6. Verfahren zur Herstellung von Verbindungen nach Anspruch 1, dadurch gekennzeichnet, dass man

a) eine Carbonsäure der Formel

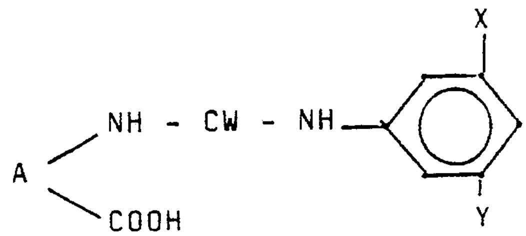

(III),

worin A, W, X und Y die obige Bedeutung haben, in Gegenwart einer starken Mineralsäure cyclisiert oder daß man

b) einen Ester der Formel

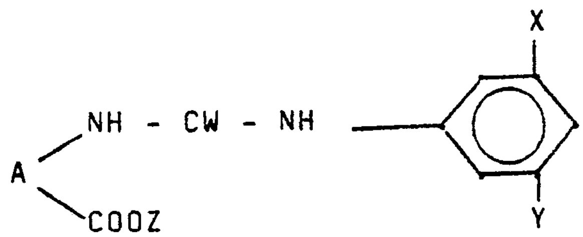

(IV),

worin A, W, X und Y die obige Bedeutung haben und Z für einen geradkettigen oder verzweigten, gegebenenfalls substituierten aliphatischen $C_1$-$C_{20}$ Rest oder einen gegebenenfalls substituierten araliphatischen Rest steht, in Gegenwart einer Base cyclisiert,

oder daß man

c) eine Aminoverbindung der Formel

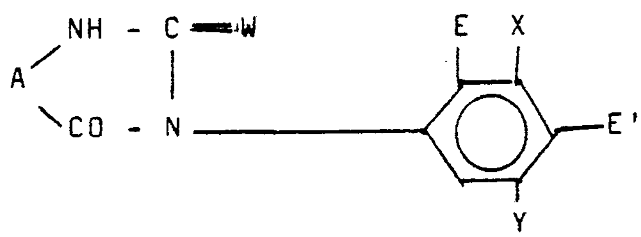

(V)

worin A, W, X und Y die obige Bedeutung haben und E und E' für Wasserstoff oder $NH_2$ stehen, wobei mindestens einer dieser beiden Reste $NH_2$ ist, entaminiert

oder daß man

d) ein Hydantoin der Formel

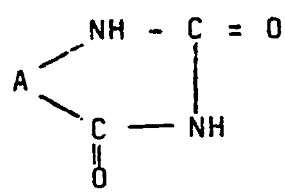

(VI)

in der A die obige Bedeutung hat,

mit einem Pyridin der Formel

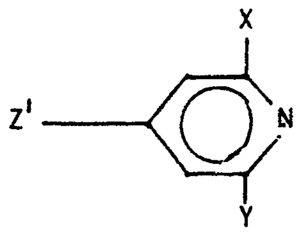

(VII),

in der X und Y die obige Bedeutung haben und Z' die Nitrogruppe oder Halogen bedeutet, in Gegenwart einer basischen Verbindung zwischen etwa 0° C und etwa 80° C umsetzt, und gegebenenfalls die erhaltenen Verbindungen in die cis- und trans-Form und/oder in die Enantiomeren auftrennt.

**7.** Verbindungen der Formel

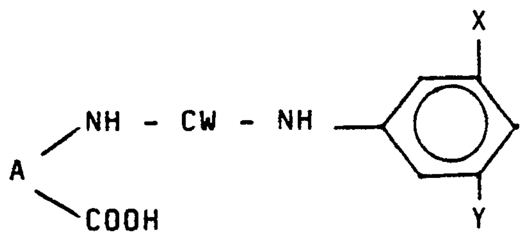

(III),

in der A, W, X und Y die obige Bedeutung haben, und ihre Salze.

**8.** Verbindungen der Formel

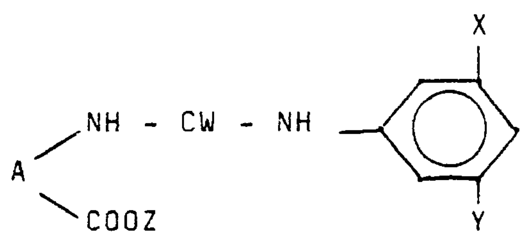

(IV),

in der A, W, X, Y und Z die obige Bedeutung haben.

**9.** Verbindungen der Formel

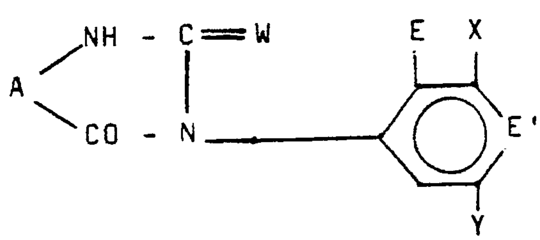

(V),

in der A, W, X, Y, E und E' die obige Bedeutung haben.

**Claims**

1. Compounds having the formula

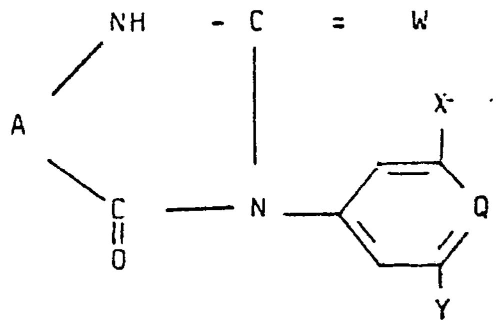

(I)

where

A represents an optionally singly or multiply bridged cycloalkane group having the formula

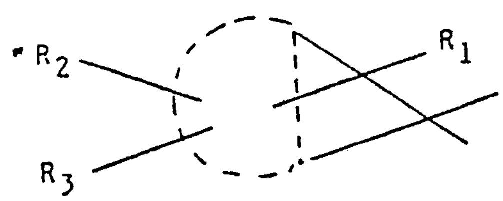

(II)

with 5 to 10 C-atoms, or the group = $CR_4R_5$,

Q represents CH or N,

$R_1$, $R_2$ and $R_3$, which may be identical or different, represent hydrogen, straight-chain or branched $C_1$-$C_4$-alkyl or straight-chain or branched $C_3$-$C_4$-alkenyl,

$R_4$ and $R_5$, which may be identical or different, represent $C_1$-$C_4$-alkyl, which may also be substituted by $C_1$-$C_4$-alkyl-O-or $C_1$-$C_4$-alkyl-S- or by a phenyl-O- or phenyl-S-group, optionally singly or multiply substituted by $C_1$-$C_4$-alkyl, $C_1$-$C_4$-alkoxy, trifluoromethyl and halogen, or represent $C_2$-$C_4$-alkenyl, $C_3$-$C_6$-cycloalkyl, which may also be substituted by a low alkyl, or represent phenyl or benzyl, optionally singly or multiply substituted by $C_1$-$C_4$-alkyl, $C_1$-$C_4$-alkoxy, trifluoromethyl and halogen, with the proviso that if $R_4$ is unsubstituted methyl, $R_5$ does not represent methyl or ethyl,

W represents oxygen, and

X and Y, which may be identical or different, represent halogen, $C_1$-$C_4$-alkyl, $C_1$-$C_4$-alkoxy or trifluoromethyl, and X also represents hydrogen

with the proviso that, if A represents an unsubstituted cycloalkyl group and Q is CH, X does not represent hydrogen.

2. Compounds according to Claim 1, where X represents hydrogen, chlorine, bromine, methyl, methoxy or trifluoromethyl and Y represents chlorine, bromine, methyl, methoxy or trifluoromethyl.

3. Herbicides, characterised in that they contain a compound according to Claim 1 or 2, together with conventional auxiliary agents and/or carriers.

4. Herbicides, characterised in that, in addition to an active substance according to Claim 1 or 2, they contain a further herbicidal active substance from the group of urea derivatives, triazine derivatives, dinitroaniline derivatives, chloro-acetanilide derivatives or thiocarbamates.

5. The use of compounds having formula I for the control of weeds and wild grasses in crops, in particular soya, maize, rice, cotton, barley, beet, potatoes, sorghum, tomatoes, onions.

**6.** A method for the preparation of compounds according to Claim 1, characterised in that

a) a carboxylic acid having the formula

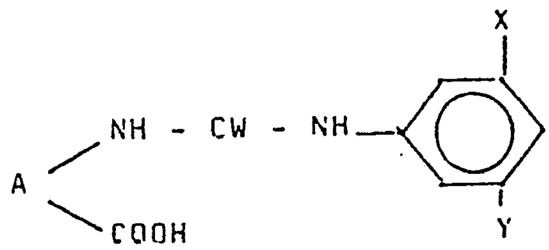

(III)

where A, W, X and Y have the above meaning, is cyclized in the presence of a strong mineral acid,

or in that

b) an ester

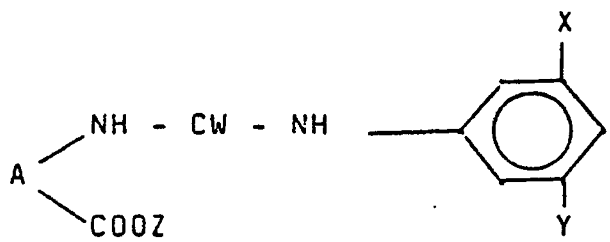

(IV)

having the formula where A, W, X and Y have the above meaning and Z represents a straight-chain or branched, optionally substituted aliphatic $C_1$-$C_{20}$ group or an optionally substituted araliphatic group, is cyclized in the presence of a base,

or in that

c) an amino compound having the formula

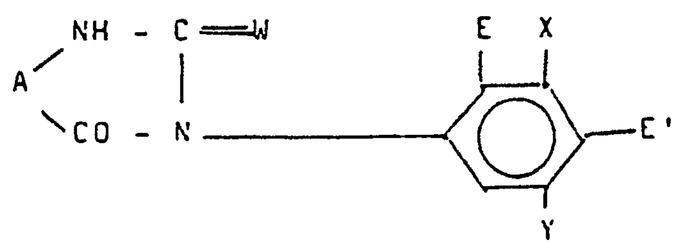

(V)

where A, W, X and Y have the above meaning and E and E' represent hydrogen or $NH_2$, at least one of the two groups being $NH_2$, is deaminated

or in that

d) a hydantoin having the formula

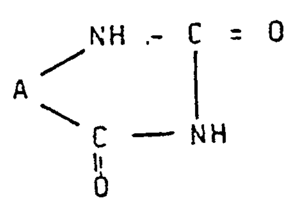

(VI)

where A has the above meaning,

is reacted with a pyridine having the formula

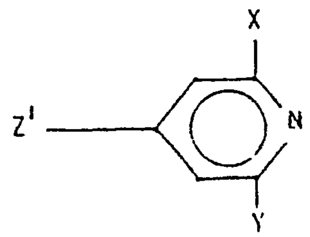

(VII)

where X and Y have the above meaning and Z' represents the nitro group or halogen, in the presence of a basic compound at temperatures between around 0° and around 80° C, and the compounds obtained are optionally separated into the cis- or trans form and/or into their enantiomers forms.

**7.** Compounds having the formula

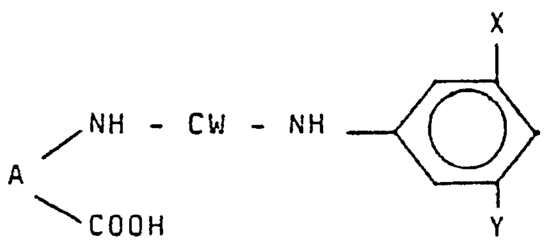

(III)

in which A, W, X and Y have the above meaning, and their salts.

**8.** Compounds having the formula

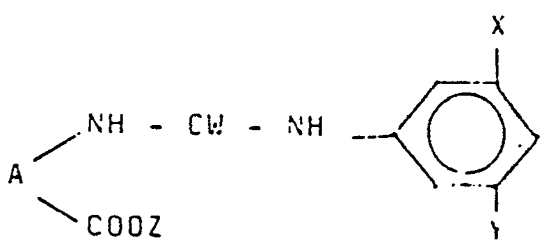

(IV)

in which A, W, X, Y and Z have the above meaning.

**9.** Compounds having the formula

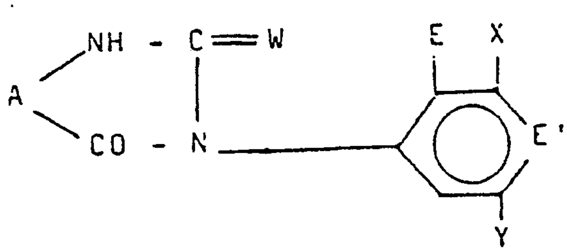

(V)

in which A, W, X, Y, E and E' have the above meaning.

**Revendications**

1. Composés de formule

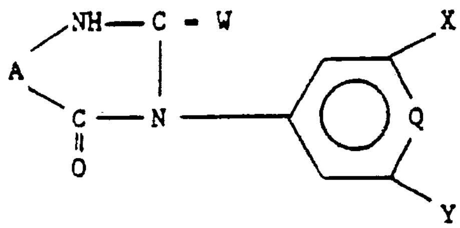

(I),

dans laquelle
A représente un radical de cycloalcane en C5-C10, éventuellement relié par un ou plusieurs ponts, et répondant à la formule

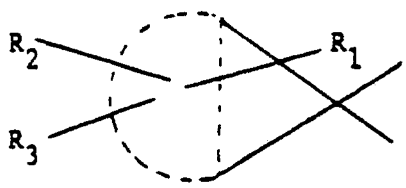

(II)

ou le groupe $=CR_4R_5$,
Q représente CH ou N,
$R_1$, $R_2$ et $R_3$, qui peuvent avoir des significations identiques ou différentes, représentent chacun l'hydrogène, un groupe alkyle en C1-C4 à chaîne droite ou ramifiée ou un groupe alcényle en C3-C4 à chaîne droite ou ramifiée,
$R_4$ et $R_5$, qui peuvent avoir des significations identiques ou différentes, représentent chacun un groupe alkyle en C1-C4, qui peut être substitué par des groupes (alkyle en C1-C4)-O- ou (alkyle en C1-C4)-S- ou par un groupe phényl-O- ou phényl-S- lui-même éventuellement mono- ou polysubstitué par des groupes alkyle en C1-C4, alcoxy en C1-C4, trifluorométhyle, un groupe alcényle en C2-C4, un groupe cycloalkyle en C3-C6 qui peut être substitué par des groupes alkyle inférieur, un groupe phényle ou benzyle éventuellement mono- ou poly-substitué par des groupes alkyle en C1-C4, alcoxy en C1-C4, trifluorométhyle, et des halogènes, sous réserve que, lorsque $R_4$ représente un groupe méthyle non substitué, $R_5$ ne peut représenter un groupe méthyle ou éthyle,
W représente l'oxygène, et
X et Y, qui peuvent avoir des significations identiques ou différentes, représentent chacun un halogène, un groupe alkyle en C1-C4, alcoxy en C1-C4 ou trifluorométhyle,
X pouvant en outre représenter l'hydrogène, sous réserve que, lorsque A représente un groupe cycloalkyle non substitué et que Q représente CH, X ne peut représenter l'hydrogène.

2. Composés selon la revendication 1, dans lesquels X représente l'hydrogène, le chlore, le brome, un groupe méthyle, méthoxy ou trifluorométhyle, Y représente le chlore, le brome, un groupe méthyle, méthoxy ou trifluorométhyle.

3. Produits herbicides caractérisés en ce qu'ils contiennent un composé selon la revendication 1 ou 2 avec des produits auxiliaires et/ou véhicules usuels.

4. Produits herbicides caractérisés en ce qu'ils contiennent, en plus d'une substance active selon la revendication 1 ou 2, une autre substance active herbicide prise dans le groupe des dérivés de l'urée, des dérivés de la triazine, des dérivés de la dinitraniline, des dérivés du chloracétanilide ou des thiocarbamates.

5. Utilisation des composés de formule I dans la lutte contre les mauvaises herbes et les végétaux adventices dans les cultures de végétaux utiles, en particulier les cultures de soya, de maïs, de riz, de

coton, d'orge, de betteraves, de pommes de terre, de millet, de tomates, d'oignons.

**6.** Procédé de préparation des composés selon la revendication 1, caractérisé en ce que
a) on cyclise un acide carboxylique de formule

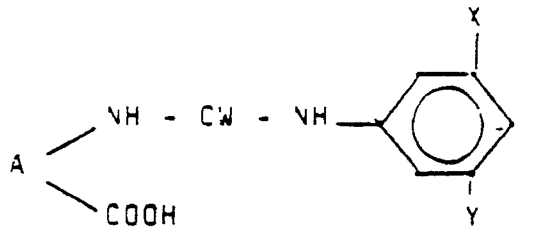

(III),

dans laquelle A, W, X et Y ont les significations indiquées ci-dessus, en présence d'un acide minéral fort, ou bien
b) on cyclise un ester de formule

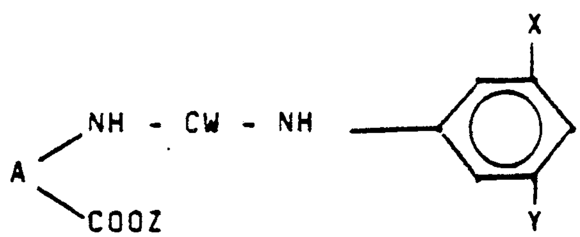

(IV),

dans laquelle A, W, X et Y ont les significations indiquées ci-dessus et Z représente un radical aliphatique à chaîne droite ou ramifiée en C1-C20 éventuellement substitué ou un radical araliphatique éventuellement substitué, en présence d'une base, ou bien
c) on soumet à désamination un dérivé aminé de formule

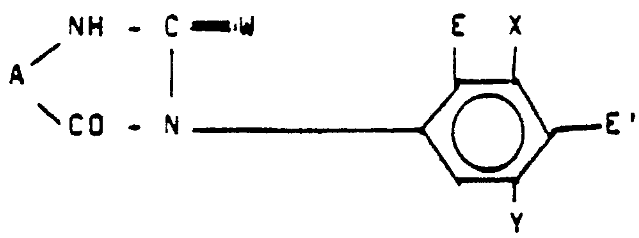

(V)

dans laquelle A, W, X et Y ont les significations indiquées ci-dessus et E et E' représentent l'hydrogène ou $NH_2$, l'un au moins de ces deux symboles représentant $NH_2$, ou bien d) on fait réagir une hydantoïne de formule

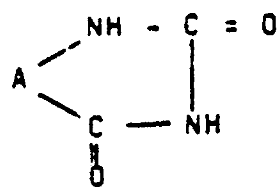

(VI)

dans laquelle A a les significations indiquées ci-dessus, avec une pyridine de formule

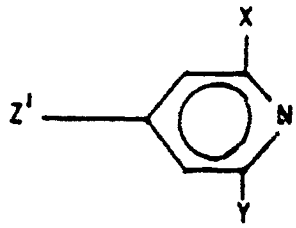

(VII),

dans laquelle X et Y ont les significations indiquées ci-dessus et Z' représente un groupe nitro ou un halogène, en présence d'un composé basique, à des températures allant de 0 à 80° C environ, et le cas échéant, on sépare les composés obtenus en la forme cis et la forme trans et/ou en les énantiomères.

**7.** Composés de formule

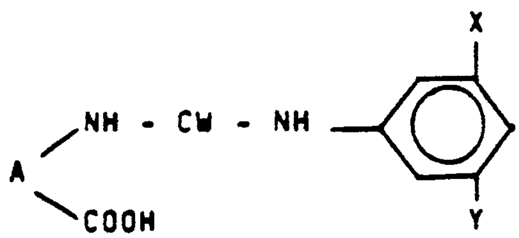

(III),

dans laquelle A, W, X et Y ont les significations indiquées ci-dessus, et leurs sels.

**8.** Composés de formule

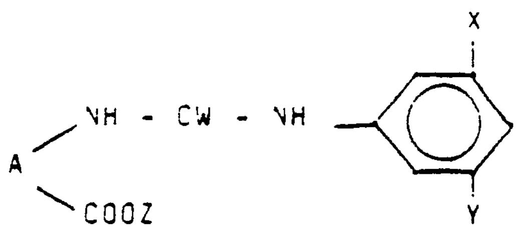

(IV),

dans laquelle A, W, X, Y et Z ont les significations indiquées ci-dessus.

**9.** Composés de formule

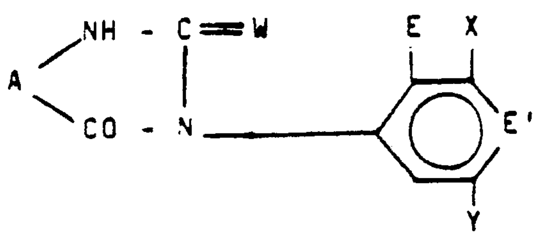

(V),

dans laquelle A, W, X, Y, E et E' ont les significations indiquées ci-dessus.